(19) Europäisches Patentamt
European Patent Office
Office européen des brevets

(11) **EP 1 305 398 B1**

(12) **FASCICULE DE BREVET EUROPEEN**

(45) Date de publication et mention
de la délivrance du brevet:
**08.06.2011 Bulletin 2011/23**

(51) Int Cl.:
***C12N 1/06*** *(2006.01)*

(21) Numéro de dépôt: **01958181.8**

(22) Date de dépôt: **26.07.2001**

(86) Numéro de dépôt international:
**PCT/FR2001/002442**

(87) Numéro de publication internationale:
**WO 2002/010333 (07.02.2002 Gazette 2002/06)**

(54) **PROCEDE DE LYSE CELLULAIRE DE PROCARYOTES OU D'EUCARYOTES OU DE LYSE CELLULAIRE SIMULTANEE DE PROCARYOTES ET D'EUCARYOTES**

VERFAHREN ZUR ZELL-LYSE VON PROKARYOTEN ODER EUKARIOTEN ODER ZUR GLEICHZEITIGEN ZELL-LYSE VON PROKARYOTEN UND EUKARIOTEN

METHOD FOR CELLULAR LYSIS OF PROKARYOTES AND EUKARYOTES OR SIMULTANEOUS CELLULAR LYSIS OF PROKARYOTES AND EUKARYOTES

(84) Etats contractants désignés:
**AT BE CH CY DE DK ES FI FR GB GR IE IT LI LU MC NL PT SE TR**

(30) Priorité: **27.07.2000 FR 0009820**

(43) Date de publication de la demande:
**02.05.2003 Bulletin 2003/18**

(73) Titulaire: **BIOMERIEUX SA**
**69280 Marcy l'Etoile (FR)**

(72) Inventeurs:
• **CLEUZIAT, Philippe**
**F-69003 Lyon (FR)**

• **INCARDONA, Sandra**
**F-69003 Lyon (FR)**
• **JAY, Corinne**
**F-69100 Villeurbanne (FR)**

(74) Mandataire: **Richaud, Fabien et al**
**Murgitroyd & Company**
**Immeuble Atlantis**
**55, Allée Pierre Ziller**
**06560 Valbonne (FR)**

(56) Documents cités:
**WO-A-00/05338    WO-A-99/15621**

EP 1 305 398 B1

## EP 1 305 398 B1

**Description**

La présente invention concerne des procédés de lyse universelle adaptés à la lyse simultanée de procaryotes et d'eucaryotes, pour le vortex mécanique.

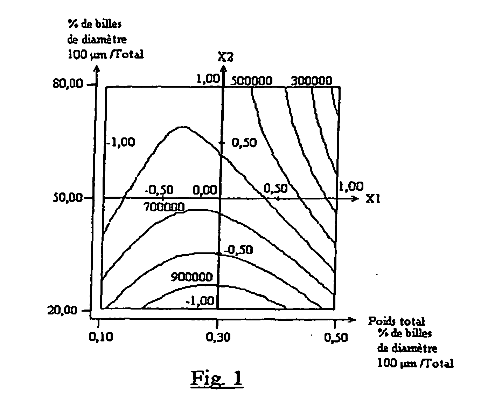

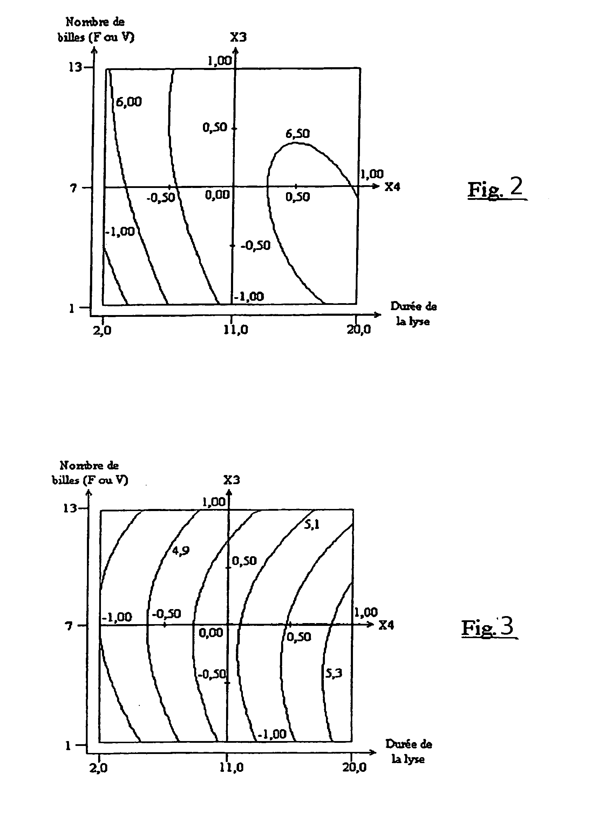

*Le vortex mécanique a déjà été décrit dans une précédente demande de brevet de la demanderesse. Il s'agit de la demande WO-A-99/15621 qui a trait à un procédé pour lyser un échantillon biologique, comprenant au moins un micro-organisme soit de type bactérie, soit de type levure. Les procédés de lyse diffèrent par le diamètre des billes qui est compris entre 90 et 150 $\mu m$, préférentiellement de 100 $\mu m$, pour les bactéries, et qui est de 500 $\mu m$ pour les levures. A noter que dans cette demande de brevet, des mélanges de billes de zirconium de 100 $\mu m$ et de 500 $\mu m$ de diamètre ont été utilisés, avec un ratio de 50 % pour chaque type de billes (voir figures 1 et 2 protocole G), afin de lyser des bactéries Staphylococcus epidermidis.*

Les résultats obtenus avec ces ratios de billes de différents diamètre, sans être mauvais, sont inférieurs à :

- ceux obtenus avec des billes de verre de 90 à 150 $\mu m$ de diamètre (protocole C),
- ceux obtenus avec des billes de verre de 100 $\mu m$ de diamètre (protocole D), ou
- ceux obtenus avec des billes de zirconium de 100 $\mu m$ de diamètre (protocole E).

Toutefois, la demanderesse a continué ces travaux dans ce domaine technique et a mis en évidence des protocoles de lyse de n'importe quels micro-organismes, que ceux-ci soient des procaryotes (bactéries) ou des eucaryotes (levures) ou un mélange d'eucaryotes et de procaryotes. Ces protocoles, dits universels, permettent une efficacité aussi importante que les lyses associées uniquement aux bactéries ou uniquement aux levures, telles que développées dans l'état de la technique ci-dessus. De manière surprenante et dans certains cas, il s'avère que ces protocoles universels sont souvent plus efficaces que les lyses de l'état de la technique.

A cet effet, la présente invention concerne un procédé de lyse cellulaire de procaryotes ou d'eucaryotes ou de lyse cellulaire simultanée de procaryotes et d'eucaryotes, par vortex mécanique, qui consiste à adapter au moins trois des paramètres suivants :

- un pourcentage en masse de billes actives de petit diamètre par rapport à des billes actives de grand diamètre inférieur ou égal à 50 %, et/ou
- une masse totale de billes lysantes, comprenant un mélange ou pas de billes de petit diamètre et/ou de billes de grand diamètre, compris entre 50 et 100 % par rapport à la masse totale de l'échantillon biologique traité, et/ou
- une durée de la lyse comprise entre 10 et 20 mn, et/ou
- un nombre de billes de verre non lysantes mais d'entraînement des billes lysantes inférieur à sept (7), et/ou
- un nombre de billes de fer non lysantes mais d'entraînement des billes lysantes compris entre cinq (5) et quinze (15).

Selon une variante préférentielle de réalisation, les billes lysantes sont en verre.

Les billes lysantes de petit diamètre sont d'un diamètre compris entre 90 et 150 $\mu m$ et préférentiellement d'environ 100 $\mu m$, et les billes lysantes de grand diamètre sont d'un diamètre compris entre 400 et 600 $\mu m$ et préférentiellement d'environ 500 $\mu m$.

Plus particulièrement, le procédé consiste à effectuer une lyse par vortex mécanique selon les paramètres suivants :

- une durée de la lyse de 11 à 20 mn, préférentiellement de 15 à 20 mn et encore plus préférentiellement de 20 mn,
- un pourcentage de billes de 100 $\mu m$ de diamètre inférieur à 50 %, préférentiellement inférieure à 30 %, et encore plus préférentiellement de 20 %,
- une masse totale de billes lysantes supérieure à 60 %, préférentiellement supérieure à 80 %, et encore plus préférentiellement de 100 % de la masse totale de l'échantillon biologique traité, et
- un nombre de billes de verre inférieur à sept (7) préférentiellement égal à un (1).

Les figures ci-jointes sont données à titre d'exemple explicatif et n'ont aucun caractère limitatif. Elles permettront de mieux comprendre l'invention.

La figure 1 représente un exemple de courbes d'isoréponses dans le cadre d'une approche MRE.
La figure 2 représente des courbes d'isoréponses dans le cadre de la lyse par vortex mécanique de levures.
Enfin, la figure 3 représente des courbes d'isoréponses dans le cadre de la lyse par vortex mécanique de bactéries.

**I. Matériel et méthodes :**

Comme exposé dans l'état de la technique ci-dessus, trois protocoles sont connus. Il s'agit de la sonication, du vortex mécanique et du vortex magnétique. Chacun d'eux a été défini pour les bactéries et pour les levures de façon indépendante. Les paramètres ainsi définis sont présentés dans le tableau 1, ils sont en rapport avec des paramètres jugés corrects de l'état de la technique. A noter que le volume des échantillons biologiques traités, que ce soit avec les protocoles de référence ou avec les protocoles selon la présente invention, est de 600 μl.

| Protocole ⟍ Facteurs | Sonic ation | | Vortex mécanique | | Vortex magnétique | |
|---|---|---|---|---|---|---|
| | bactéries | levures | bactéries | levures | bactéries | levures |
| Diamètre(μm) et quantité (g) de billes actives | 100 et 0,4 | 500 et 0,3 | 100 et 0,6 | 500 et 0,5 | 100 et 0,6 | 500 et 0,5 |
| Nombre billes de verre (3 mm de diamètre) | / | / | 6 | | / | / |
| Nombre billes de fer (2 mm de diamètre) | / | / | 10 | | 10 | |
| Durée de lyse | 15 mn | | 2 mn | 12 mn | 10 mn | 12 mn |

## Tableau 1 : Protocoles de lyse selon l'état de la technique appelés protocoles de référence

Les billes actives dites « lysantes » sont constituées des billes qui permettent la lyse des micro-organismes. Il s'agit des billes de petits diamètres (100 ou 500 μm). Il convient de noter que les billes de verre de 100 μm de diamètre ont été obtenues chez Masteau et Lamarié, Paris, France, sous la référence Via 1, et que les billes de verre de 500 μm de diamètre proviennent de la société Sigma, Saint Louis, Missouri, Etats-Unis d'Amérique, sous la référence 8772.
Par contre, les autres billes de verre (3 mm de diamètre) et de fer (2 mm de diamètre), utilisées pour les vortex mécanique et magnétique, ont pour objectif de mettre en mouvement les billes actives lors d'une agitation. Elles sont appelées billes passives ou actives indirectement, via les billes lysantes ci-dessus définies. Les billes de verre de 3 mm de diamètre ont été achetées chez Prolabo (Fontenay-sous-Bois, France) sous la référence 00680032, et c'est Bennk Elektronic (Norderstadt, Allemagne) qui a fourni les billes de fer de 2 mm de diamètre, sous la référence 050-330.
A noter qu'il est possible d'utiliser des billes de fer recouvertes d'une matière biologiquement inerte, telle que plastique ou verre, afin d'isoler le fer et d'éviter son relargage qui est souvent incompatible avec les opérations d'amplification ultérieures d'acides nucléiques libérés par la lyse.
Les résultats obtenus avec les procédés selon l'invention seront par la suite comparés avec les résultats obtenus par l'intermédiaire des procédés de référence ci-dessus décrits.
L'intérêt d'un protocole universel de lyse est de permettre la détection, après lyse, de micro-organismes responsables d'infections de fluides biologiques stériles (liquide céphalo-rachidien, urine et sang), sans savoir à priori s'il s'agit de micro-organismes procaryotes (bactéries) ou eucaryotes (levures).

**A) Méthologie de la Recherche Expérimentale :**

Une approche par la Méthologie de la Recherche Expérimentale (MRE) a été utilisée pour le vortex mécanique.
L'étude MRE comprend deux étapes. Dans un premier temps et pour différents facteurs, une zone optimale propre aux levures et une zone optimale propre aux bactéries ont été définies. Cette zone optimale est définie comme celle donnant une efficacité de lyse équivalente ou supérieure à celle obtenue avec le protocole de référence.

Dans un deuxième temps et si possible, une zone dite de compromis, correspondant à l'intersection de la zone optimale bactérie avec la zone optimale levures, est définie.

La MRE est une approche mathématique qui consiste à modéliser un phénomène biologique, en l'occurrence la lyse, par une équation mathématique (souvent une équation mathématique polynomiale du second degré) du type :

$$R = a\,X + b\,Y + c\,X^2 + d\,Y^2 + e\,XY + f,$$

où

- R est la réponse expérimentale traduisant l'effficacité de la lyse,
- X et Y sont des facteurs influençant la lyse comme la durée de la lyse et la taille des billes, et
- a, b, c, d, e et f sont des coefficients.

Cette équation peut-être représentée sous forme de courbes dites d'isoréponses permettant de visualiser comment varie la réponse en fonction des valeurs prises par les différents facteurs. La figure 1 donne un exemple de courbes d'isoréponses.

Selon cette figure 1, deux facteurs sont étudiés. Il s'agit du poids total (X1) de billes lysantes de 100 et 500 $\mu$m de diamètre et le pourcentage en masse de billes de 100 $\mu$m de diamètre par rapport au total de billes lysantes de 100 et 500 $\mu$m de diamètre(X2); la réponse étudiée correspond à un nombre d'unités de fluorescence RLU, c'est-à-dire Relative Light Unit. Les courbes représentées relient les points du domaine expérimental donnant une réponse identique. On voit sur ce graphe que la réponse maximale obtenue dans le domaine expérimental est de 900 000 unités de fluorescence, et pour obtenir cette réponse, il faut que X1 soit compris entre 0,2 et 0,4 et X2 soit proche de 20.

L'obtention du modèle mathématique nécessite la réalisation de plusieurs expériences définies par des matrices dites « matrices composites », bien décrites dans les documents suivants :

- Box and Wilson (1951) « On the expérimental atteignement of optimal conditions » Journal of the Royal Statistical Society, B, 13, p. 1-45.
- Feneuille, Mathieu, Phan-Tan-Lun (1978) « Méthodologie de la Recherche Expérimentale. Introduction, outils mathématiques, études des surfaces de réponse, matrices de mélanges ; méthodes du simplex ; Université d'Aix-Marseille 3 - Publication du LMRE.

B) Réponse étudiée :

L'efficacité de la lyse est quantifiée en mesurant la quantité d'ARN 16S et 23S (pour les bactéries) et d'ARN ribosomal (pour les levures) libérée par hybridation avec une sonde chemiluminescente. Il s'agit d'une mesure HPA (Hybridization Protection Assay). Il s'agit d'une technique de la société Gen-Probe bien décrite dans l'article de Norman C. Nelson, Mark A. Reynolds et Lyle J. Arnold Jr : « Détection of Acridinium Esters by Chemiluminescence » Nonisotropic probing, Blotting and Sequencing (1995) 391-428. Des informations sur cette technique peuvent également être trouvées dans le brevet US-A-6,004,745.

On a utilisé deux sondes l'une développée par Gen Probe et commercialisée sous le nom Mycoplasma Tissue Culture NI (MTC-NI), voir leur prospectus : 104574 Revendication. A. Comme il est précisé en page 3 dans le chapitre « Performance Characteristics », cette sonde peut détecter une large variété de micro-organismes à Gram négatif et à Gram positif, sans toutefois croiser avec des cibles eucaryotes.

Une seconde sonde pour les levures a été utilisée. Il est ainsi possible d'utiliser une sonde telle que celles décrites par :

- J. F. Hindler, S. Kozen, D. A. Bruckner dans leur article : « Application of an rRNA Probe Matrix for Rapid Identification of Bacteria and Fungi in Routine Blood Cultures » N° 1557, et
- D. D. Fuller, T. E. Davis dans l'article : « Comparison of rRNA Probe Matrix to Conventional Methods for Rapid Identification of Clinically Significant Bacteria and Fungi Recovered from Blood Cultures Specimens » N°1558, de la session 154.D, page 221, Laboratory Tests for Diagnosing Infections ; Methods for susceptibility testing.
- Abstracts book, ICAAC - Sept. 26-29, 1999 - San Francisco - USA - Deux posters présentent l'utilisation d'une sonde universelle permettant la détection de l'ensemble des espèces de champignons (moisissures et levures)

C) Facteurs étudiés :

Les facteurs que l'on peut étudier sont :

- le poids total de billes actives (en g),
- le pourcentage en masse de billes lysantes de 100 $\mu$m,
- la durée de la lyse (en mn),
- le nombre de billes de verre de 3 mm additionnelles, et
- le nombre de billes de fer de 2 mm additionnelles.

D) Souches de levure et bactérie utilisées :

La souche de levure retenue pour l'étude est *Candida krusei* ayant comme numéro de collection bioMérieux: API n° 9604191. La souche de bactérie est *Mycobacterium fortuitum* déposée auprès de l'ATCC sous le numéro : 49403.

La lyse est réalisée sur 600 $\mu$l d'une suspension à 0,5 Mac Farland (densitomètre bioMérieux).

## II. Résultats préliminaires selon les procédés de références :

A) Résultats montrant que les billes de 100 $\mu$m sont plus efficaces que les billes de 500 $\mu$m pour lyser les bactéries :

Le tableau 2 ci-dessous représente les résultats obtenus où toutes les unités sont en HPA comme dans les tableaux suivants numérotés 3 à 10.

Tableau 2 : Etude comparative entre les billes de 100 $\mu$m et les billes de 500 $\mu$m pour la technique de lyse des bactéries par vortex mécanique

|  | Billes actives de diamètre 100 $\mu$m | | Billes actives de diamètre 500 $\mu$m | |
|---|---|---|---|---|
|  | Valeurs brutes | Moyenne | Valeurs brutes | Moyenne |
| Vortex mécanique | 105 263<br>96 142<br>97 875<br>102 771 | **100 513** | 39 624<br>41 879<br>50 584<br>43 697 | **43 946** |

On remarque aisément que les billes de 100 $\mu$m sont plus efficaces que les billes de 500 $\mu$m.

B) Résultats comparant la performance des billes de 500 $\mu$m et de 100 $\mu$m pour lyser les levures :

Le tableau 3 ci-dessous est représentatif des résultats obtenus.

Tableau 3 : Etude comparative entre les billes de 100 $\mu$m et les billes de 500 $\mu$m pour la technique de lyse des levures par vortex mécanique

|  | Billes actives de diamètre 100 $\mu$m | | Billes actives de diamètre 500 $\mu$m | |
|---|---|---|---|---|
|  | Valeurs brutes | **Moyenne** | Valeurs brutes | **Moyenne** |
| Vortex mécanique | 1 392 101<br>1 511 603<br>1 493 588<br>1 128 468<br>1 061 514 | **1 317 454** | 2 464 220<br>2 598 929<br>2 234 830<br>2 695 394<br>2 833 394 | **2 565 353** |

Les billes de 500 $\mu$m sont plus efficaces que les billes de 100 $\mu$m pour lyser les levures. Un protocole universel nécessite un mélange de billes de 100 et 500 $\mu$m.

**III. Résultats de la lyse par vortex mécanique selon la présente invention :**

Quatre paramètres sont utilisés, il s'agit du pourcentage de billes de 100 μm de diamètre, de la masse totale de billes lysantes de 100 et de 500 μm du nombre de billes de verre de 3 mm additionnelles et de la durée de la lyse (en minutes).

A) Vortex mécanique et levures :

Le protocole de référence utilise une masse totale de billes lysantes de 0,3 g, un pourcentage de billes de 100 μm de diamètre de 0%, 6 billes de verre et 10 billes de fer, ainsi qu'un temps de lyse de 12 mn. Les valeurs HPA obtenues sont de 1 944 115, 2 213 485 et 2 158 958 soit une moyenne de 2 105 519. Le tableau 4 met en évidence les résultats obtenus.

Tableau 4 : Etude comparative entre la technique de référence et des techniques pouvant répondre aux critères de la présente invention pour la lyse par vortex mécanique des levures

| Pourcentage de billes de 100 μm de diamètre | | | 20 % | | 50 % | | 80 % | |
|---|---|---|---|---|---|---|---|---|
| Masse de billes lysantes | Nombre de billes verre | Durée de la lyse | Valeurs brutes | Moyenne | Valeurs brutes | Moyenne | Valeurs brutes | Moyenne |
| 0,1 g | 1 | 2 mn | 173 382 198 469 191 565 | 187 805 | | NT = non testé | 53 771 55 591 51 464 | 53 609 |
| | 13 | 2 mn | 383 782 875 488 647 615 | 635628 | | NT | 566 150 631 271 479 612 | 559 011 |
| | 1 | 20 mn | 1 499 619 696 373 262 854 | 819 615 | t | NT | 569 050 590 737 679 410 | 613 066 |
| | 13 | 20 mn | 1 009 588 1 472 753 1 101 463 | 1 194 601 | | NT | 852 141 1 041 371 1 217 872 | 1 037 128 |
| | 7 | 11 mn | | | 731 593 543160 718173 | 664 309 | | NT |

(suite)

| Masse de billes lysantes | Nombre de billes verre | Durée de la lyse | Pourcentage de billes de 100 μm de diamètre | | | | | |
| --- | --- | --- | --- | --- | --- | --- | --- | --- |
| | | | 20 % | | 50 % | | 80 % | |
| | | | Valeurs brutes | Moyenne | Valeurs brutes | Moyenne | Valeurs brutes | Moyenne |
| 0,35 g | 7 | 11 mn | 1 772 021 1 817 222 1 878 805 | 1 822 683 | | NT | 1 342 768 1 740 035 1 391 889 | 1 491 564 |
| | 1 | 11 mn | | NT | 896 736 1 329 122 975 083 | 1 066 980 | | NT |
| | 13 | 11 mn | | NT | 1 429 173 1 439 611 1 588 337 | 1 485 707 | | NT |
| | 7 | 2 mn | | NT | 323 207 271 399 388 199 | 327 602 | | NT |
| | 7 | 20 mn | | NT | 2 655 347 2 620 069 2 321 318 | 2 532 245 | | NT |
| | 7 | 11 min | | NT | 1 519 280 1 503 048 1 375 956 | 1 466 095 | | NT |
| 0,6 g | 1 | 2 mn | 509 952 278 641 349 239 | 379277 | | NT | 60 273 105 730 76 940 | 80 981 |
| | 13 | 2 mn | 1 070 276 794 021 725 931 | 863 409 | | NT | 234 974 467 140 260 342 | 320 819 |
| | 1 | 20 mn | 2 856 141 3 176 777 2 825 049 | 2 952 656 | | NT | 1 767 239 1 412 744 1 682 354 | 1 620 779 |
| | 13 | 20 mn | 1 826 409 2 156 630 2 017 243 | 2 000 094 | | NT | 1 500 483 816 002 372 770 | 896 418 |
| | 7 | 11 mn | | NT | 2 257 042 1 868 266 2 104 986 | 2 076 765 | | NT |

La figure 2 met en évidence les courbes d'isoréponses ainsi obtenues. L'analyse des courbes montre que le facteur le plus influent sur la réponse est la durée. Pour maximiser la lyse, il faut une durée de la lyse de 20 mn, un pourcentage de billes de 100 μm de diamètre inférieur à 50 %,une masse totale de billes lysantes supérieure à 0,35 g et un nombre de billes de verre inférieur à sept (7).

Un exemple de courbes d'isoréponses est donné pour un poids total de 0,5 g et un pourcentage de billes de 100 μm de diamètre de 20%.

On démontre que pour augmenter la réponse, il faut une durée de lyse supérieure à 11 mn, un pourcentage de billes de 100 μm de diamètre inférieure à 50 %, une masse totale de billes lysantes supérieure à 0,35 g et un nombre de billes de verre inférieur à 7.

B) Vortex mécanique et bactéries :

Le protocole de référence utilise une masse totale de billes lysantes de 0,6 g, un pourcentage de billes de 100 μm de diamètre de 100%, 6 billes de verre et 10 billes de fer, et ce pendant un temps de lyse de 2 mn ; les valeurs HPA trouvées sont de 107 406, 124 609 et 138 405, soit une moyenne de 123 473.

Le tableau 5 met en évidence les résultats obtenus.

Tableau 5 : Etude comparative entre la technique de référence et des techniques pouvant répondre aux critères de la présente invention pour la lyse par vortex mécanique des bactéries

| Pourcentage de billes de 100 μm de diamètre | | | 20 % | | 50 % | | 80 % | |
|---|---|---|---|---|---|---|---|---|
| Masse de billes lysantes | Nombre de billes verre | Durée de la lyse | Valeurs brutes | Moyenne | Valeurs brutes | Moyenne | Valeurs brutes | Moyenne |
| 0,1 g | 1 | 2 mn | 52 553 74 120 70 675 | 65 783 | | NT | 63 184 58 656 58 185 | 60 008 |
| | 13 | 2 mn | 14 521 119 746 39 993 | 58 087 | | NT | 41 586 54 066 40 387 | 45 346 |
| | 1 | 20 mn | 342 532 192 353 422 974 | 319 286 | | NT | 143 231 141 178 141 240 | 141 883 |
| | 13 | 20 mn | 180 946 152 548 | 166 747 | | NT | 62 916 33 591 89 886 | 62 131 |
| | 7 | 11 mn | NT | NT | 95 433 89 768 81 290 | 88 830 | | NT |

(suite)

| Pourcentage de billes de 100 μm de diamètre | | | 20 % | | 50 % | | 80 % | |
|---|---|---|---|---|---|---|---|---|
| Masse de billes lysantes | Nombre de billes verre | Durée de la lyse | Valeurs brutes | Moyenne | Valeurs brutes | Moyenne | Valeurs brutes | Moyenne |
| 0,35 g | 7 | 11 mn | 96 363 82 168 138 530 | 105687 | | NT | 164 967 133 405 146 659 | 148 344 |
| | 1 | 11 mn | | NT | 182 124 134 177 97 015 | 137 772 | | NT |
| | 13 | 11 mn | | NT | 79 136 58 570 71 041 | 69 582 | | NT |
| | 7 | 2 mn | | NT | 68 180 55 805 42 366 | 55 450 | | NT |
| | 7 | 20 mn | | NT | 326 925 229 725 142 933 | 233 194 | | NT |
| | 7 | 11 mn | | NT | 103 348 90 163 96 770 | 96 760 | | NT |
| 0,6 g | 1 | 2 mn | 54 466 49 842 | 52 154 | | NT | 108 236 82 578 130 014 | 106 943 |
| | 13 | 2 mn | 80 796 63 431 | 72 113 | | NT | 102 979 162 136 104 830 | 123 315 |
| | 1 | 20 mn | 275 653 153 372 249 164 | 226 063 | | NT | 255 286 265 434 223 440 | 248 053 |
| | 13 | 20 mn | 189 947 107 754 112 081 | 136 594 | | NT | 176 619 183 820 185 692 | 182 044 |
| | 7 | 11 mn | | NT | 131 318 121 488 153 508 | 135 438 | | NT |

La figure 3 met en évidence les courbes d'isoréponses ainsi obtenues. L'analyse des courbes montre que le facteur le plus actif sur la lyse est la durée. Pour augmenter la réponse, il faut une durée maximale de lyse de 20 mn et un faible nombre de billes de verre. De plus, la réponse est un peu meilleure avec un pourcentage de billes de 100 μm de diamètre faible, proche de 20 %, et une masse totale de billes lysantes proche de 0,6 g, ces 2 facteurs ayant une moindre influence.

Un exemple de courbes est donné pour une masse totale de billes lysantes fixée à 0,6 g et un pourcentage de billes de 100 μm de diamètre fixé à 20 %. C'est le cas sur la figure 5.

Les courbes montrent que la durée de lyse a un effet important sur la réponse, la réponse maximum est obtenue pour une durée de 20 mn. Le nombre de billes de verre a moins d'effet sur la lyse, une seule bille de verre étant suffisante.

C) Choix d'un protocole universel de lyse par vortex mécanique :

Le protocole universel est donc défini par les paramètres suivants

- durée de la lyse de 20 mn,
- pourcentage de billes de 100 $\mu$m de diamètre = 20 %,
- masse totale de billes lysantes = 0,5 g, et
- nombre de billes de verre égal à une (1), avec un diamètre de sensiblement 3 mm.

Dans ces conditions, on obtient les résultats surprenants suivant :

- la moyenne obtenue pour les bactéries est de 226 063 alors qu'elle est de 123 473 pour le protocole de référence utilisant 100 % de billes de 100 $\mu$m,
- la moyenne obtenue pour les levures est de 2 952 6S6 alors qu'elle est de 2 105 519 pour le protocole de référence utilisant 100 % de billes de 500 $\mu$m.

Le procédé associé à ce protocole de lyse universelle consiste donc à effectuer un vortex mécanique selon les paramètres suivants :

- une durée de la lyse de 11 à 20 mn, préférentiellement de 15 à 20 mn et encore plus préférentiellement de 20 mn,
- un pourcentage de billes de 100 $\mu$m de diamètre inférieur à 50 %, préférentiellement inférieure à 30 %, et encore plus préférentiellement de 20 %,
- une masse totale de billes lysantes supérieure à 60 %, préférentiellement supérieure à 80 %, et encore plus préférentiellement de 100 % de la masse totale de l'échantillon biologique traité, et
- un nombre de billes de verre inférieur à sept (7) préférentiellement égal à une (1).

**IV. Conclusion :**

Il est démontré que pour la technique de vortex mécanique, il est possible de définir un protocole universel, c'est-à-dire, capable de lyser des cellules procaryotes et/ou eucaryotes avec une efficacité identique et souvent supérieure à celle obtenue avec les protocoles dits de référence

**Revendications**

1.  Procédé de lyse cellulaire de micro-organismes procaryotes ou eucaryotes ou de lyse cellulaire simultanée de micro-organismes procaryotes et eucaryotes, consistant à effectuer un vortex mécanique en adaptant les paramètres suivants:

     - un pourcentage en masse de billes lysantes de petit diamètre par rapport à des billes lysantes de grand diamètre inférieur ou égal à 50 %,
     - une masse totale de billes lysantes, comprenant un mélange de billes de petit diamètre et de billes de grand diamètre, compris entre 50 et 100 % par rapport à la masse totale de l'échantillon biologique traité,
     - une durée de la lyse comprise entre 10 et 20 mn, et
     - un nombre de billes d'entraînement des billes lysantes, en verre, inférieur à sept (7),

     dans lequel les billes lysantes, de petit diamètre sont d'un diamètre compris entre 90 et 150 $\mu$m et préférentiellement d'environ 100 $\mu$m, et les billes lysantes de grand diamètre sont d'un diamètre compris entre 400 et 600 $\mu$m et préférentiellement d'environ 500 $\mu$m.

2.  Procédé, selon la revendication 1, **caractérisé en ce que** les billes lysantes sont en verre.

3.  Procédé, selon la revendication 1 ou 2, **caractérisé en ce qu'**il consiste à effectuer un vortex mécanique selon les paramètres suivants ;

     - une durée de la lyse de 11 à 20 mn, préférentiellement de 15 à 20 mn et encore plus préférentiellement de 20 mn,
     - un pourcentage de billes de 100 $\mu$m de diamètre inférieur à 50 %, préférentiellement inférieure à 30 %, et encore plus préférentiellement de 20 %,

- une masse totale de billes lysantes supérieure à 60 %, préférentiellement supérieure à 80 %, et encore plus préférentiellement de 100 % de la masse totale de l'échantillon biologique traité, et
- un nombre de billes de verre inférieur à sept (7) préférentiellement égal à un (1).

**Claims**

1. Method of cellular lysis of procaryotic or eucaryotic microorganisms or of simultaneous cellular lysis of procaryotic and eucaryotic microorganisms, consisting in creating a mechanical vortex by adapting the following parameters:

   - a percentage by mass of lysing beads of small diameter relative to lysing beads of large diameter less than or equal to 50%,
   - a total mass of lysing beads, including a mixture of beads of small diameter and of beads of large diameter of between 50 and 100% relative to the total mass of the biological sample treated,
   - a duration of lysis of between 10 and 20 min, and
   - a number of beads for driving the lysing beads, made of glass, less than seven (7),

   in which the lysing beads of small diameter are of a diameter of between 90 and 150 $\mu$m and preferably of approximately 100 $\mu$m, and the lysing beads of large diameter are of a diameter of between 400 and 600 $\mu$m and preferably of approximately 500 $\mu$m.

2. Method, according to claim 1, **characterised in that** the lysing beads are made of glass.

3. Method, according to claim 1 or 2, **characterised in that** it consists in creating a mechanical vortex in accordance with the following parameters:

   - a duration of lysis of 11 to 20 min, preferably of 15 to 20 min and still more preferably of 20 min,
   - a percentage of beads of 100 $\mu$m diameter less than 50%, preferably less than 30%, and still more preferably of 20%,
   - a total mass of lysing beads greater than 60%, preferably greater than 80%, and still more preferably of 100% of the total mass of the biological sample treated, and
   - a number of glass beads less than seven (7) preferably equal to one (1).

**Patentansprüche**

1. Ein Verfahren zur Zell-Lyse von prokaryotischen oder eukaryotischen Mikroorganismen oder zur gleichzeitigen Zell-Lyse von prokaryotischen oder eukaryotischen Mikroorganismen, bestehend aus dem Erzeugen eines mechanischen Vortex durch das Anpassen der folgenden Parameter:

   - eines Massenanteils von lysierenden Perlen mit kleinem Durchmesser im Vergleich zu lysierenden Perlen mit großem Durchmesser von kleiner oder gleich 50 %,
   - einer Gesamtmasse von lysierenden Perlen, umfassend eine Mischung aus Perlen mit kleinem Durchmesser und Perlen mit großem Durchmesser von zwischen 50 und 100 % im Vergleich zur Gesamtmasse der behandelten biologischen Probe,
   - einer Dauer der Lyse von 10 bis 20 min und
   - einer Anzahl von Perlen zum Antreiben der lysierenden Perlen, aus Glas, von weniger als sieben (7),

   wobei die lysierenden Perlen mit kleinem Durchmesser einen Durchmesser von zwischen 90 und 150 $\mu$m und vorzugsweise von ungefähr 100 $\mu$m aufweisen und die lysierenden Perlen mit großem Durchmesser einen Durchmesser von zwischen 400 und 600 $\mu$m und vorzugsweise von ungefähr 500 $\mu$m aufweisen.

2. Verfahren gemäß Anspruch 1, **dadurch gekennzeichnet, dass** die lysierenden Perlen aus Glas sind.

3. Verfahren gemäß Anspruch 1 oder 2, **dadurch gekennzeichnet, dass** es daraus besteht, einen mechanischen Vortex nach den folgenden Parametern zu erzeugen:

   - einer Dauer der Lyse von 11 bis 20 min, vorzugsweise von 15 bis 20 min und noch bevorzugter von 20 min,

- einem Prozentsatz von Perlen mit einem Durchmesser von 100 $\mu$m von weniger als 50 %, vorzugsweise weniger als 30 % und noch bevorzugter 20 %,
- einer Gesamtmasse von lysierenden Perlen von mehr als 60 %, vorzugsweise mehr als 80 % und noch bevorzugter 100 % der Gesamtmasse der behandelten biologischen Probe und
- einer Anzahl von Glasperlen von weniger als sieben (7), vorzugsweise gleich eins (1).

Fig. 1

Fig. 2

Fig. 3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 6004745 A

**Littérature non-brevet citée dans la description**

- **Box ; Wilson.** On the expérimental atteignement of optimal conditions. *Journal of the Royal Statistical Society,* 1951, vol. B, 13, 1-45
- **Feneuille ; Mathieu ; Phan-Tan-Lun.** *Méthodologie de la Recherche Expérimentale,* 1978
- **Norman C. Nelson ; Mark A. Reynolds ; Lyle J. Arnold Jr.** Détection of Acridinium Esters by Chemiluminescence. *Nonisotropic probing, Blotting and Sequencing,* 1995, 391-428
- **J. F. Hindler ; S. Kozen ; D. A. Bruckner.** *Application of an rRNA Probe Matrix for Rapid Identification of Bacteria and Fungi in Routine Blood Cultures*
- Comparison of rRNA Probe Matrix to Conventional Methods for Rapid Identification of Clinically Significant Bacteria and Fungi Recovered from Blood Cultures Specimens. **D. D. Fuller ; T. E. Davis.** Comparison of rRNA Probe Matrix to Conventional Methods for Rapid Identification of Clinically Significant Bacteria and Fungi Recovered from Blood Cultures Specimens. Laboratory Tests for Diagnosing Infections, vol. 154.D, 221
- ICAAC. 26 Septembre 1999